(19) Europäisches Patentamt

European Patent Office

Office européen des brevets

(11) Veröffentlichungsnummer: **0 326 985**

**A2**

(12) **EUROPÄISCHE PATENTANMELDUNG**

(21) Anmeldenummer: 89101533.1

(51) Int. Cl.⁴: **A61L 2/20**

(22) Anmeldetag: 30.01.89

(30) Priorität: 03.02.88 US 151878

(43) Veröffentlichungstag der Anmeldung:
09.08.89 Patentblatt 89/32

(84) Benannte Vertragsstaaten:
AT BE CH DE ES FR GB GR IT LI LU NL SE

(71) Anmelder: MESSER GRIESHEIM INDUSTRIES INC.
2400 Boulevard of the Generals
Valley Forge, PA 19482(US)

(72) Erfinder: Brahmbhatt, Sudhir R.
6237 Sage Drive
Macungie Pennsylvania 18062(US)

(74) Vertreter: Roesner, Werner
MESSER GRIESHEIM GmbH Patentabteilung
Lärchenstrasse 137 Postfach 83 00 48
D-6230 Frankfurt/Main 83(DE)

(54) **Verfahren und Einrichtung zur Rückgewinnung von Sterilisiergas.**

Verfahren und Einrichtung zur Rückgewinnung mindestens einer Komponente eines Sterilisiergases, wobie das Sterilisiermittel und das Verdünnungsgas in einem Verflüssiger getrennt werden und das Sterilisiermittel flüssig anfällt und mindestens eine Komponente nach entsprechender Aufbereitung entweder wieder verwendet oder einem Lagerbehälter zugeführt wird.

EP 0 326 985 A2

## Verfahren und Einrichtung zur Rückgewinnung von Sterilisiergas

(57) Die Erfindung umfaßt ein Verfahren und eine Einrichtung zur Rückgewinnung einer oder mehrere Komponenten eines Sterilisiergases. Typischerweise ist das Sterilisiergas Ethylenoxid, gemischt mit einem verhältnismäßig inerten Verdünnungsgas wie einem Halogenkohlenstoff. Das aus einer Sterilisierkammer ausgeströmte Sterilisiergas wird in einem Verflüssiger auf tiefe Temperaturen abgekühlt, so daß das Ethylenoxid und das Verdünnungsgas verflüssigt werden. Die zurückbleibenden gasförmigen Verunreinigungen, wie z.B. Luft, werden aus dem Verflüssiger entfernt, filtriert und in die Atmosphäre ausströmen gelassen. Die verflüssigte Mischung von Ethylenoxid und Verdünnungsgas wird periodisch aus dem Verflüssiger abgelassen und zur Entfernung von Verunreinigungen filtriert. Diese flüssige Mischung kann dann für die spätere Verwendung in die Komponenten, aus denen sie besteht, getrennt werden. Die flüssige Mischung kann auch zur Herstellung einer Sterilisiergasmischung, die in die Sterilisierkammer zurückgeführt wird, verdampft und je nach Bedarf mit frischem Ethylenoxid versetzt werden. In einer bevorzugten Ausführung überwacht ein Gaschromatograph den Ethylenoxidgehalt der aus der Einrichtung ausströmenden Verunreinigungen, so daß ein unerwünschtes Ausströmen von Ethylenoxid aus der Einrichtung verhindert werden kann. Der Gaschromatograph kann auch die Konzentration von Ethylenoxid in dem Gas überwachen, das in den Sterilisator zurückgeleitet wird, so daß die Zusammensetzung dieses zurückströmenden Gases geregelt werden kann. Die Erfindung nach das Sterilisationsverfahren sicherer und wirtschaftlicher als Verfahren nach dem bisherigen Stand der Technik.

Bei der Erfindung handelt es sich um eine Einrichtung und ein Verfahren zur sicheren und wirtschaftlichen Behandlung eines Sterilisiergases. Insbesondere bietet die Erfindung ein Mittel zur Rückgewinnung einer oder mehrerer Komponenten eines Sterilisiergases, insbesondere eines solchen Sterilisiergases, das Ethylenoxid und einen Halogenkohlenstoff als Verdünnungsgas enthält.

Ethylenoxid (ETO) findet als Sterilisiermittel breite Verwendung. Es tötet Bakterien wirkungsvoll nach einer Einwirkung von bis zu mehreren Stunden. Es ist besonders nützlich zum Sterilisieren von wärme- oder feuchtig-keitsempfindlichen Artikeln. Wegen seines großen Ausbreitungsvermögens kann es zum Sterilisieren von Kombinationen mehrerer Produkte, die sich innerhalb von verschweißten Kunststoffolien, Versandkartons und Behältern befinden, verwendet werden.

Wegen des verhältnismäßig hohen Preises von ETO ist es üblich, das Gas mit einer verhältnismäßig reaktionsträgen Substanz wie Dichlordifluormethan ($CCl_2F_2$) (Freon) zu verdünnen. In der Regel wird eine Sterilisierungsgasmischung mit einem Massenanteil von etwa 12 % ETO und 88 % Freon verwendet. Andere Verdünnungsgase, beispielsweise andere Arten von Halogenkohlenstoffen oder $CO_2$, können auch verwendet werden.

Vor kurzem wurde festgestellt, daß ETO ein ernstes Gesundheitsrisiko darstellt. Es ist bekanntermaßen ein Mutagen und steht unter dem Verdacht, ein Carcinogen zu sein.

In der Vergangenheit war es allgemein üblich, nach Beendigung des Sterilisationsverfahrens die Sterilisiergasmischung aus dem Bereich, in dem die Sterilisation erfolgte, auszutreiben und die Mischung einfach in die Atmosphäre austreten zu lassen. Dieses Verfahren ist wegen der bekannten Umweltgefahren von ETO nicht mehr möglich. Darüber hinaus wird von den zur Verdünnung des Sterilisiergases verwendeten Fluorkohlenstoffen jetzt angenommen, daß sie die Ozonschicht der Atmosphäre schädigen.

Sowohl ETO als auch Freon ausströmen zu lassen, ist nicht nur umweltschädlich, sondern auch eine wirtschaftliche Verschwendung, da beide Stoffe verhältnismäßig teuer sind. Die vorliegende Erfindung ermöglicht es, beide Komponenten zurückzugewinnen und eine oder beide wiederzuverwenden, wodurch die schädliche Belastung minimiert wird.

Ein Beispiel für die Rückgewinnung eines Sterilisiergases wird im US-Patent Nr. 4,249,917 beschrieben. Das Patent zeigt ein Verfahren zur Trennung von Sterilisiergas, bei dem die Abtrennung von ETO chemisch erfolgt, indem die Sterilisiergasmischung mit einem ETO absorbierenden organischen Lösungsmittel in Kontakt gebracht wird. Ein Nachteil des geschilderten Verfahrens ist das Erfordernis, Füllkörpersäulen und auch das ETO-absorbierende Lösungsmittel bereitzustellen. Diese Elemente verteuern das Verfahren. Überdies bildet das Lösungsmittel, von dem das ETO absorbiert wird, Glykol, das selbst ein gefährlicher Stoff ist und nur nach genehmigten Verfahren, und unter zusätzlichen Kosten, beseitigt werden darf.

Die vorliegende Erfindung offenbart ein Verfahren und eine Einrichtung die viel wirtschaftlicher sind als der bisherige Stand der Technik. Durch die Rückführung des ETO werden die Gesamtkosten für das zur Sterilisation benötigte ETO stark gesenkt. Das System gemäß der vorliegenden Erfindung minimiert auch die Emission von gefährlichen Stoffen wie ETO und Freon in die Atmosphäre.

Gemäß der vorliegenden Erfindung wird Sterilisiergas aus einer Sterilisierkammer abgelassen.

Das abgelassene Sterilisiergas enthält ETO und Freon sowie andere Verunreinigungen, wie Luft und etwas polymerisiertes ETO. Das abgelassene Gas wird in einen Tieftemperaturverflüssiger geleitet. Eine Tieftemperaturflüssigkeit, wie flüssiger Stickstoff, kühlt den Verflüssiger und verflüssigt das ETO und das Freon. Die flüssige Mischung wird aus dem Verflüssiger entfernt und zur Verminderung oder Beseitigung des polymerisierten ETO filtriert. Gleichzeitig werden die gasförmigen Verunreinigungen im Sterilisier gas, die nicht verflüssigt werden, aus dem Verflüssiger hinausgeleitet, durch ein geeignetes Filter geschickt und in due Atmosphäre abgelassen.

In einer ersten Alternative wird das Sterilisiergas in die Sterilisierkammer zurückgeleitet. In dieser Alternative wird die flüssige Mischung von ETO und Freon verdampft und eine Probe des Dampfes mit einem Gaschromatographen analysiert. Zusätzliches ETO wird je nach Bedarf hinzugegeben, um den Sollwert der Konzentration von ETO in der Mischung aufrechtzuerhalten. Es können auch zusätzliche Mengen an ETO-Freon-Mischung hinzugegeben werden, um das infolge von Leckage verlorene Freon zu ersetzen. Die wiedergewonnene Mischung wird dann in die Sterilisierkammer geleitet.

In einer zweiten Alternative wird die flüssige Mischung von ETO und Freon in eine Destillationssäule transportiert, wo das ETO und das Freon getrennt werden. Auf diese Art und Weise ermöglicht die Erfindung die Rückgewinnung einer oder mehrerer Komponenten des Sterilisiergases, auch wenn diese Komponenten nicht sofort wiederverwendet werden.

Gemäß der Erfindung können die oben beschriebenen Alternativen entweder einzeln oder zusammen verwendet werden.

Bei beiden obigen Alternativen kann der Gaschromatograph auch zur Messung des restlichen gasförmigen ETO in der in die Atmosphäre ausströmenden Mischung verwendet werden. Wenn der ETO-Anteil zu groß wird, liefert der Chromatograph ein Signal, das bewirkt, daß das ausströmende Gas durch ein anderes, sauberes Filter geleitet wird. Die Erfindung minimiert deshalb die ETO-Menge, die man in die Atmosphäre austreten läßt.

Ziel der Erfindung ist ein Verfahren und eine Einrichtung zur Behandlung eines Sterilisiergases.

Ein weiteres Ziel ist ein Verfahren und eine Einrichtung zur Rückgewinnung einer oder mehrerer Komponenten eines Sterilisiergases.

Ein weiteres Ziel ist ein Verfahren und eine Einrichtung zur Wiederverwendung einer oder mehrerer Komponenten eines Sterilisiergases.

Ein weiteres Ziel ist ein Verfahren und eine Einrichtung wobei die Einrichtung verschiedene Verunreinigungen aus dem Sterilisiergas entfernt.

Ein weiteres Ziel ist es, die Menge an Ethylenoxid zu senken, die man in die Atmosphäre entweichen läßt, und die Gefahren infolge der Verwendung von Ethylenoxid in einem Sterilisiergas zu minimieren.

Ein weiteres Ziel ist es, die Menge an Fluorkohlenstoffen wie Freon zu senken, die man in die Atmosphäre entweichen läßt.

Ein weiteres Ziel ist es, die Menge des in einem Sterilisiersystem benötigten Ethylenoxids und Freons zu senken.

Ein weiteres Ziel ist es, die Kostengünstigkeit und Sicherheit der Sterilisation von Artikeln mit Ethylenoxid zu verbessern.

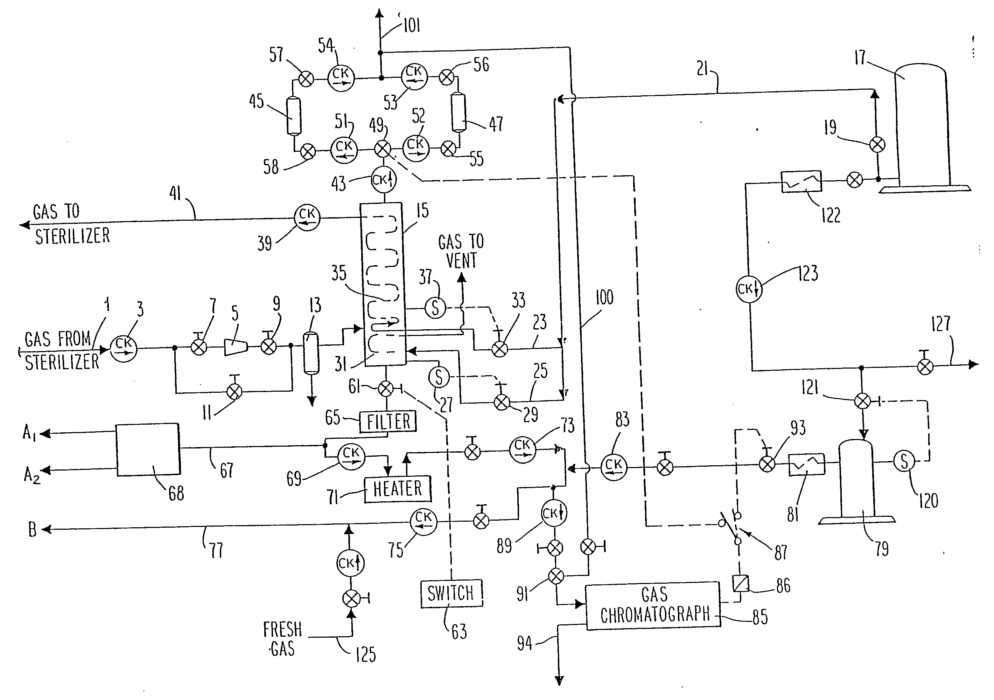

Weitere Ziele und Vorteile der Erfindung ergeben sich aus der Beschreibung der Erfindung und der Zeichnung.

Die Figur ist eine Prinzipskizze der Einrichtung zur Rückgewinnung und Wiederverwendung eines Sterilisiergases gemäß der vorliegenden Erfindung.

Wie in der Figur gezeigt, tritt Gas, das zum Sterilisieren von Gegenständen in einer Sterilisierkammer (nicht gezeigt) verwendet wurde, in das System gemäß der vorliegenden Erfindung durch die Leitung 1 ein, wobei es durch das Rückschlagventil 3 strömt. Das durch Leitung 1 strömende Sterilisiergas (auch "abgelassenes Sterilisiergas" gennant) enthält im allgemeinen Ethylenoxid (ETO), Freon und andere Verunreinigungen, einschließlich polymerisiertem ETO und Luft. Das Sterilisiergas in Leitung 1 hat typischerweise einen Überdruck von etwa 10 - 15 psi (ca. 7 - 10 kPa) und eine Temperatur von ca. 130 °F (ca. 50 - 60 °C). Wenn jedoch der Überdruck des Sterilisiergases in der Sterilisierkammer näher bei null Pa liegt, so ist es erforderlich, seinen Druck zu erhöhen, so daß das Gas ausreichend durch das System gedrückt wird. Der Druck für diesen Zweck kann durch Verwendung des Kompressors 5 erhalten werden. Ferner muß man, wenn man eine Mischung von verflüssigten ETO und Freon liefern will, die zur Einleitung in einen Hochdruckzylinder geeignet ist, das Gas noch stärker komprimieren.

Liegt der Druck im System nahe bei Atmosphärendruck, so ist est im allgemeinen erforderlich, das Sterilisiergas abzulassen, indem man ein anderes Gas wie Stickstoff bei einem höheren Druck in den Sterilisator drückt, um das Sterilisiergas auszutreiben.

Der Kompressor ist also nicht unbedingt erforderlich; ist der Druck in der Sterilisierkammer größer als der Betriebsdruck des Systems, so kann der Kompressor weggelassen werden. Die Ventile 7 und 9 erleichtern das Entfernen des Kompressors aus dem System und seine Ersetzung, und Ventil 11 bietet einen Umleitungsweg für das abgelassene Sterilisiergas, wenn der Kompressor im System nicht vorhanden ist oder wenn er vorhanden, aber augenblicklich nicht in Gebrauch ist.

Das abgelassene Gas kann durch den Trockner 13 geschickt werden, der ein Kondensator sein kann, der Wasserrückstände aus dem Gas entfernt. Der Trockner ist auch nicht unbedingt erforderlich.

Das abgelassene Gas strömt dann weiter in den Verflüssiger 15. Das Kühlmittel für den Verflüssiger 15 ist eine Tieftemperaturflüssigkeit. Die Tieftemperaturflüssigkeit ist vorzugsweise flüssiger Stickstoff, aber es könnten andere Tieftemperaturflüssigkeiten verwendet werden. Flüssiger Stickstoff aus dem Vorratstank 17 fließt durch das Ventil 19 und durch Leitung 21. Der flüssige Stickstoff wird in zwei Ströme aufgeteilt, von denen einer in die Leitung 23 geht und der andere in die Leitung 25 eintritt.

Der Sensor 27 ist zur Messung der Temperatur des Inhalts des unteren Bereichs des Verflüssigers 15 angeschlossen. Wie die gestrichelte Linie anzeigt, ist der Sensor 27 zur Betätigung von Ventil 29 angeschlossen. Bei normalen Betrieb des Systems enthält der untere Bereich des Verflüssigers eine flüssige Mischung von ETO und Freon. Sensor 27 und Ventil 29 haben den Zweck sicherzustellen, daß das ETO am Boden des Verflüssigers eine Flüssigkeit bleibt. Ermittelt also der Sensor, daß die Temperatur dieser flüssigen Mischung zu hoch ist, so bewirkt der Sensor, daß sich das Ventil 29 öffnet und somit mehr flüssigen Stickstoff einläßt. Hat die Temperatur den gewünschten Wert erreicht, so bewirkt der Sensor die Schließung des Ventils. Flüssiger Stickstoff fließt durch Leitung 31, verdampft infolge der von ihm aufgenommenen Wärme und tritt, wie gezeigt, nach außen aus. Der die Leitungen 25 und 31 durchströmende Stickstoff kommt nicht mit dem Inhalt des Verflüssigers in Berührung.

Der flüssige Stickstoff aus dem Tank 17 tritt auch in die Leitung 23 ein und fließt durch das Ventil 33 in die Leitung 35, die sich vorzugsweise durch den gesamten Verflüssiger erstreckt, mit Ausnahme des unteren Bereichs. Der Stickstoff in der Leitung 35 senkt die Temperatur des abgelassenen Sterilisierens ab, so daß das ETO und das Freon verflüssigt und diese Komponenten von den Verunreinigungen im Sterilisiergas getrennt werden. Die Stickstoffleitung 35 wird nur benötigt, wenn die Sterilisierkammer entleert wird und Gas durch den Verflüssiger strömt. Im Gegensatz hierzu wird der Stickstoff in der Leitung 31 immer (oder zumindest über einen viel größeren Teil der gesamten Zeit) benötigt, weil es wichtig ist, daß die Komponenten am Boden des Verflüssigers flüssig bleiben. Der Sensor 37 überwacht die Temperatur im mittleren und/oder oberen Berich des Verflüssigers 15 und betätigt das Ventil 33, um je nach Bedarf mehr Stickstoff in die Leitung 35 einzulassen. Die Leitung 35 wird schematisch in einer Serpentinenform gezeigt, wobei die Leitung durch den größten Teil des mittleren und oberen Bereichs des Verflüssigers verläuft. Der Stickstoff in Leitung 35 fließt durch das Rückschlagventil 39 in die Leitung 41, wo er für die Verwendung im Sterilisator zur Verfügung steht. Dieser Stickstoff wird, nachdem er im Verflüssiger Wärme aufgenommen hat, verdampft und kann zum Entfernen des Sterilisiergases aus der Sterilisierkammer verwendet werden.

Wenn das ausgetretene Sterilisiergas im Verflüssiger gekühlt wird, so werden ETO und Freon verflüssigt, und die gasförmigen Verunreinigungen, wie Luft, verlassen den Verflüssiger durch das Rückschlagventil 43. Das austretende Gas/Gasgemisch wird je nach Stellung des Dreiwegventils 49 durch eines der Filter 45 oder 47 geleitet. Die Betätigung des Dreiwegventils wird später beschrieben. Die Rückschlagventile 51, 52, 53 und 54 stellen die richtige Richtung der Strömung sicher, und die Ventile 55, 56, 57 und 58 erleichtern die Entfernung der Filter 45 und 47 aus dem System und ihre Ersetzung.

Die Filter 45 und 47 haben übliche Filtereinsätze. Die Einsätze sollten von genügender Größe und Kapazität sein, so daß jeder von ihnen dazu fähig ist, das gesamte aus dem Verflüssiger austretende Gas/Gasgemisch zu verarbeiten. Die Filter entfernen auch ETO-Rückstände aus dem austretenden Gas.

Nach dem Druchgang durch eines der Filter 45 oder 47 tritt das Gas in die Atmosphäre aus. Aufgrund der Abtrennung des ETO und des Freons von dem abgelassenen Sterilisiergas im Verflüssiger und aufgrund der weiteren Wirkung der Filter ist das nach außen austretende Gas praktisch frei von ETO und Freon.

Periodisch ist es erwünscht, das verflüssigte ETO und Freon aus dem unteren Bereich des Verflüssigers zu entfernen und diese Komponenten wieder im Sterilisationsverfahren zu verwenden. Das Ventil 61 wird durch Schalter 63 betätigt, wobei die flüssige Mischung infolge der Schwerkraft aus dem Verflüssiger abfließt.

Alternativ, wenn das System bei einem höheren Druck betrieben wird, braucht das Filter nicht unter dem Verflüssiger zu liegen, und die Flüssigkeit kann durch Druck anstatt durch Schwerkraft entfernt werden. Die Flüssigkeit wird zur Beseitigung von polymerisiertem ETO aus dem Flüssigkeitsstrom durch das Filter 65 geleitet. Das Filter 65 kann ein übliches Filter sein. Diese Filter sind gewöhnlich aus rostfreiem Stahl hergestellt und für das Filtrieren von flüssigem ETO gedacht. Die flüssige Mischung von ETO und Freon braucht nicht ganz so kalt zu sein wie der sie kühlende flüssige Stickstoff, sie sollte aber ca. -200 °F (ca. -100 °C) sein.

Die das Filter 65 verlassende Flüssigkeit kann durch die Leitung 67 geschickt und in die Füllkörpersäule 68 geleitet werden, in der das ETO vom Freon getrennt wird. Das ETO und das Freon werden dann durch die Leitungen A1 und A2 abgegeben.

Der durch das Filter 65 gehende Flüssigkeitsstrom kann auch durch das Rückschlagventil 69 in die Heizung 71 fließen. Die Heizung 71 ist vorzugsweise eine elektrische Heizung und dient zum Verdampfen der Flüssigkeit. Diese verdampfte Flüssigkeit strömt durch die Rückschlagventile 73 und 75 in die Leitung 77, die zum Sterilisator zurückführt. Das die Heizung 71 verlassende Gas wird im allgemeinen nicht die gewünschten Anteile an ETO und Freon (vorzugsweise 12 % ETO und 88 % Freon) haben. Etwas ETO geht gewöhnlich aufgrund von Polymesrisation und aufgrund er Absorption des ETO in dem Produkt, das sterilisiert wurde, verloren. In geringerem Ausmaß kann infolge von Leckage ein Verlust an Freon eintreten. Es ist deshalb erforderlich, die Anteile durch Zugabe einer geeigneten Menge von ETO und, gelegentlich, durch Zugabe einer frischen Mischung von ETO und Freon einzustellen.

Zusätzliches flüssiges ETO ist im Tank 79 gespeichert. Der Druck im Tank 79 wird von dem Sensor 120 überwacht, der das Ventil 121 regelt. Flüssiger Stickstoff aus dem Tank 17 wird in dem Verdampfer 122 verdampft und strömt durch das Rückschlagventil 123, druch das Ventil 121 und in den Tank 79. In Tank 79 wird genug Stickstoff geleitet, um den Druck im Tank aufrechtzuerhalten. Weil der Stickstoff inert und in dem ETO unlöslich ist, wird wenig óder kein Stickstoff in das System eintreten. In dem Maße jedoch, in dem kleine Mengen von Stickstoff in das System eintreten, wird es aufgrund der Reaktionsträgheit des Stickstoffs keine Beeinträchtigungen geben.

Wird das System bei einem verhältnismäßig geringen Überdruck, wie etwa 10 - 15 psi (ca. 7 - 10 kPa) betrieben, und die Flüssigkeit in Tank 79 bei einem Überdruck von beispielsweise 60 psi (ca. 40 kPa) gespeichert, so wird die Flüssigkeit einen Druckverlust erleiden, wenn sie durch das Ventil 93 hindurchgeht. Dieser Druckverlust wird dazu führen, daß die Flüssigkeit verdampft. Wenn das System jedoch bei einem höheren Überdruck, beispielsweise 40 - 50 psi (ca. 35 - 35 kPa) betrieben, so ist die Druckdifferenz zwischen dem Tank und dem System nicht groß, und der Druckabfall reicht möglicherweise nicht dafür aus, zur Verdampfung zu führen. Im letzteren Falle wird der Verdampfer 81 verwendet. Der Verdampfer 81 ist also nicht unbedingt erforderlich; er wird nur verwendet, wenn der Druckabfall nicht zur Verdampfung des ETO ausreicht. Ob nun der Verdampfer vorhanden ist oder nicht, strömt das ETO durch das Rückschlagventil 83 in die Leitung 131, die schließlich in die Leitung 77 führt.

Die Strömung des gasförmigen ETO wird durch den Gaschromatographen 85 bestimmt. Der Chromatograph ist zur Durchführung von Stichproben am Gas des Ausgangsstroms, d.h. des durch das Rückschlagventil 73 und die Leitung 77 fließenden Gases, angeschlossen. Die Gasprobe strömt durch das Rückschlagventil 89, durch das Dreiwegventil 91 und in den Chromatographen. Die Probe wird dann durch Leitung 94 aus dem Chromatographen abgelassen.

Der Chromatograph ist an die Schaltvorrichtung 87 angeschlossen, die symbolisch als einpoliger Umschalter eingezeichnet ist. Wenn die Schaltvorrichtung 87 in der durch die gepunktete Linie gezeigten Stellung ist, ist der Chromatograph mit dem Regler 93 verbunden, der ein Ventil ist, das regelbar veränderliche Mengen von ETO durchströmen läßt. Der Meßwert des Chromatographen, der von der ETO-Menge im Probenstrom abhängt, bestimmt so die ETO-Menge, die zum Ausgangsstrom zugesetzt wird. Wenn der ETO-Gehalt des Ausgangsstroms zu niederig ist, öffnet sich der Regler 93 und erlaubt so, daß mehr ETO in den Strom eintritt. Umgekehrt schließt sich der Regler, bei zu hohem ETO-Gehalt.

Der Ausgang des Chromatographen kann mit dem Signalumwandler 86 verbunden werden, der den Chromatographenausgang von einem ékektrischen Signal in ein pneumatisches Signal umwandeln könnte, wenn der Regler 93 pneumatisch betätigt wird. Oder, wenn der Regler 93 elektrisch betätigt wird, könnte der Signalumwandler 86 ein Wandler oder ein Signalformer zur Umwandlung des Chromatographenausgangssignals in eine besser verwendbare Form sein. Der Signalumwandler 86 könnte in Fällen weggelassen werden, wo es nicht erforderlich ist, das Signal von Chromatographen zu wandeln oder auf andere Art zu bearbeiten.

Der Gaschromatograph ist auch, über Leitung 100, mit der Ausgangsleitung 101 verbindbar, durch die Gas aus dem System ausströmt. Dieses ausströmende Gas tritt durch das Dreiwegventil 91 in den Chromatographen ein. Das Dreiwegventil 91 ist so konstruiert, daß es entweder das aus dem Rückschlagventil 89 strömende Gas oder das durch die Leitung 100 strömende Gas in den Chromatographen eintreten läßt, aber nicht beide gleichzeitig. Ist das Dreiwegventil 91 so eingestellt, daß das Gas durch die Leitung 100 in den Chromatographen eintreten kann, so muß der Schalter 87 auch in die durch die durchgezogene Linie gezeigte Richtung gestellt sein. Das heißt, wenn der Chromatograph Gasproben von der Leitung 100 nimmt, ist er auch operativ mit dem Dreiwegventil 49 verbunden. Ist die ETO-Menge in Leitung 100 zu groß, so bedeutet das, daß das Filter (entweder 45 oder 47, je nachdem welches gerade verwendet wird) ersetzt werden muß. Der Chromatograph erzeugt dann ein Signal, das zu einer Änderung der Stellung des Dreiwegventils 48 führt, d.h. zu einer Umleitung des Ausgangsstroms vom Verflüssiger 15 in das Filter, das nicht verwendet worden war. Wenn also gerade Filter 45 verwendet wird und die ETO-Konzentration in der Entlüftungsleitung zu groß wird, so leitet das Ventil den Strom um zu Filter 47. Wird gerade Filter 47 verwendet, so leitet das Ventil den Strom um zu Filter 45. Das bisher verwendete Filter kann dann ersetzt werden, während das System seinen Betrieb mit dem anderen Filter fortsetzt.

Der Gaschromatograph wird von der Schaltvorrichtung 87 periodisch umgeschaltet, so daß er seine Funktionen nacheinander ausführen kann. Zu diesen Funktionen gehört das Analysieren des Ausgangsstroms des Sterilisiergases, das gleich in den Sterilisator zurückgeleitet werden soll, und die Überwachung des Gases, das in die Atmosphäre abgelassen wird, um sicher zu sein, daß der ETO-Anteil innerhalb der gewünschten Grenzen liegt. Der Chromatograph kann nur jeweils eine dieser Aufgaben erfüllen. Es ist auch möglich, wenngleich mit höheren Kosten verbunden, zwei Chromatographen vorzusehen, die beide kontinuierlich betrieben werden und den oben beschriebenen Funktionen fest zugeordnet sind.

Bei normalen Betrieb geht bei jedem Sterilisationszyklus infolge der Bildung von ETO-Polymeren und der Absorption durch das zu sterilisierende Produkt etwas ETO verloren. Die Polymere werden durch das Filter 65 beseitigt und sind für das System verloren. Das Freon geht, infolge von Lekkage, viel langsamer verloren. Das verlorengegangene ETO wird, wie oben erörtert, aus Tank 79 wieder aufgefüllt. Zur Ersetzung des Freons, das durch Leckage verlorengehen kann, kann man nach wiederholtem Betrieb eine frische Mischung von gasförmigem ETO und Freon in Leitung 77 einlassen, wie durch die Leitung 125 angedeutet ist. Das ETO in der Leitung 125 kann aus dem Tank 79 abgeleitet werden oder aus einer getrennten Versorgung kommen.

Die Leitung 127 bietet eine alternative Quelle von gasförmigem Stickstoff, der zum Entleeren der Sterilisierkammer verwendet werden kann. Der Stickstoff in Leitung 127, der im Verdampfer 122 verdampft wurde, ist verhältnismäßig warm und kann sogar Zimmertemperatur haben. In manchen Anwendungen wird es vorgezogen, daß der zum Entleeren dieser Kammer verwendete gasförmige Stickstoff Zimmertemperatur hat. Im Gegensatz hierzu ist der gasförmige Stickstoff in der Leitung 41, der zuerst nach dem Durchgang durch den Verflüssiger verdampft wurde, noch recht kalt.

Die vorliegende Erfindung liefert deshalb zwei verschiedene Arten von Produkten. Erstens ist sie fähig zur Rückgewinnung der einzelnen Komponenten des Sterilisiergases, d.h. ETO und Freon, und zur getrennten Speicherung dieser Komponenten zur späteren Verwendung in einem Sterilisationsverfahren oder für andere Zwecke. Zweitens ist sie fähig zur Reinigung und Rückführung des Sterilisiergases, mit der Zugabe von mehr ETO, je nach Bedarf. Die Erfindung kann so betrieben werden, daß sie eine dieser Funktionen oder beide ausführt. Mit anderen Worten: der mit der Leitung 67 zusammenhängende Teil und der mit der Leitung 77 zusammenhängende Teil des Systems können beide als Wahlmöglichkeiten betrachtet werden. Welche Alternative auch verwendet wird, die in die Atmosphäre austretenden Mengen von ETO und Freon werden minimiert und die insgesamt verbrauchten Mengen gesenkt.

Obwohl die Erfindung in Hinsicht auf eine spezifische Verkörperung beschrieben wurde, versteht es sich, daß die Erfindung modifiziert werden kann. Wie oben gesagt, sind viele der einzelnen Komponenten nicht unbedingt erforderlich und brauchen nur in bestimmten Anwendungen verwendet zu werden. Die besonderen für den Bau der Filter verwendeten Werkstoffe, das zum Kühlen des Sterilisiergases verwendete Mittel und das Mittel zur Versorgung mit zusätzlichem ETO können alle modifiziert werden. Die Erfindung ist auch nict begrenzt auf die Verwendung von ETO als Sterilisiergas oder Freon als Verdünnungsgas. Andere Sterilisier- und Verdünnungsmittel können im Rahmen der Erfindung verwendet werden.

## Ansprüche

1. Einrichtung für die Behandlung eines Sterilisiergases, wobei das Sterilisiergas ein Sterilisiermittel und ein inertes Verdünnungsmittel enthält, gekennzeichnet dadurch:

(a) Vorrichtung (128) zum Transport des Sterilisiermittels und des Verdünnungsgases von einer Sterilisierkammer zu einer Verflüssigungsvorrichtung (15),

(b) Vorrichtung 31, 35 zum Kühlen der Verflüssigungsvorrichtung (15) zur Verflüssigung des Sterilisiermittels und des Verdünnungsgases und zur Trennung des Sterilisiergases von gasförmigen Verunreinigungen mit denen das Sterilisiergas gemischt ist,

(c) Vorrichtung (129) zum Abtransport der abgetrennten gasförmigen Verunreinigungen von der Verflüssigungsvorrichtung (15) und zum Filtrieren (45, 47) und Ablassen (101) der gasförmigen Verunreinigungen,

(d) Vorrichtung (130) zum Abtransport der Flüssigkeit von der Verflüssigungsvorrichtung (15) und zum Filtrieren (65) der gennanten Flüssigkeit,

(e) Vorrichtung (71) zum Verdampfen der filtrierten Flüssigkeit zur Herstellung einer Menge gereinigten Sterilisiermittels und Verdünnungsgases,

(f) Vorrichtung (131) zur Leitung des gereinigten Sterilisiermittels und Verdünnungsgases in eine Rückleitung (77) die mit der Sterilisierkammer verbunden ist,

(g) Vorrichtung (132) zum Einstellen der Menge des Sterilisiermittels und des Verdünnungsgases in der Rückleitung (77) und

(h) Vorrichtung (125) zum Hinzufügen einer Menge frischen Verdünnungsgases zum Sterilisiermittel in der Rückleitung (77).

2. Einrichtung gemäß Anspruch 1, dadurch gekennzeichnet, daß das Sterilisiermittel Ethylenoxid ist.

3. Einrichtung gemäß Anspruch 1, dadurch gekennzeichnet, daß das Verdünnungsgas Dichlordifluormethan ist.

4. Einrichtung gemäß Anspruch 1, dadurch gekennzeichnet, daß die Kühlvorrichtung (15) eine Versorgung (17) mit einer Tieftemperaturflüssigkeit und eine Leitungsvorrichtung (21, 23, 25) enthält, welche die Versorgung (17) und die Verflüssigungsvorrichtung (15) verbindet.

5. Einrichtung gemäß Anspruch 4, dadurch gekennzeichnet, daß die Verflüssignungsvorrichtung (15) einen unteren und einen oberen Bereich hat und worin es mindestens zwei Leitungen (31, 35) gibt, welche die Tieftemperaturflüssigkeit enthalten, wobei die Leitung (31) dazu bestimmt ist, Tieftemperaturflüssigkeit durch den unteren Bereich der Verflüssigungsvorrichtung (15) zu leiten und die Leitung 35 dazu bestimmt ist, Tieftemperaturflüssigkeit durch den oberen Bereich zu leiten.

6. Einrichtung gemäß Anspruch 1, dadurch gekennzeichnet, daß die Einstellungsvorrichtung (132) eine Vorrichtung (85) zum Messen der Menge des Sterilisiergases in der Rückleitung (77) enthält, wobei die Meßvorrichtung (85) operativ verbunden ist mit einer Ventilvorrichtung (87) zur Regelung der Strömung zusätzlichen Sterilisiermittels aus einer Versorgung (79) mit Sterilisiergas in die Rückleitung (77).

7. Einrichtung zur Rückgewinnung einer oder mehrerer Komponenten eines Sterilisiergases, wobei das Sterilisiergas ein Sterilisiermittel und ein inertes Verdünnungsgas enthält, gekennzeichnet durch:

(a) Vorrichtung (128) zur Förderung des Sterilisiermittels und des Verdünnungsgases von einer Sterilisierkammer zu einer Verflüssigungsvorrichtung (15),

(b) Vorrichtung 31, 35) zum Kühlen des Gases in der Verflüssigungsvorrichtung (15) zur Verflüssigung des Sterilisiermittels und des Verdünnungsgases und zur Trennung des Sterilisiergases von gasförmigen Verunreinigungen, mit denen das Sterilisiergas gemischt ist,

(c) Vorrichtung (129) zum Abtransport der abgetrennten gasförmigen Verunreinigungen von der Verflüssigungsvorrichtung (15) und zum Filtrieren (45, 47) und Ablassen (101) der genannten gasförmigen Verunreinigungen,

(d) Vorrichtung (130) zum Abtransport der Flüssigkeit aus der Verflüssigungsvorrichtung (15) und zum Filtrieren (65) der gennanten Flüssigkeit und

(e) Vorrichtung (68) zur Abtrennung des verflüssigten Sterilisiermittels vom verflüssigten Verdünnungsgas.

8. Einrichtung gemäß Anspruch 7, dadurch gekennzeichnet, daß das Sterilisiermittel Ethylenoxid ist.

9. Einrichtung gemäß Anspruch 7, dadurch gekennzeichnet, daß das Verdünnungsgas Dichlordifluormethan ist.

10. Einrichtung gemäß Anspruch 7, worin die Kühlvorrichtung (15) eine Versorgung (17) mit einer Tieftemperaturflüssigkeit enthält und eine Leitungsvorrichtung (21, 23, 25) welche die Versorgung (17) mit der Verflüssigungsvorrichtung (15) verbindet.

11. Einrichtung gemäß Anspruch 10, dadurch gekennzeichnet, daß die Verflüssigungsvorrichtung (15) einen unteren und einen oberen Bereich hat, und in der es mindestens zwei Leitungen (31, 35) gibt, welche die Tieftemperaturflüssigkeit enthalten, wobei die Leitung (31) dazu bestimmt ist, Tieftemperaturflüssigkeit durch den unteren Bereich der Verflüssigungsvorrichtung (15) zu leiten und die Leitung (35) dazu bestimmt ist, Tieftemperaturflüssigkeit durch den oberen Bereich zu leiten.

12. Einrichtung zur Rückgewinnung einer oder mehrerer Komponenten eines Sterilisiergases, wobei das Sterilisiergas ein Sterilisiermittel und ein Verdünnungsgas enthält, das Sterilisiergas aus einer Sterilisierkammer ausgetrieben wurde und die Einrichtung gekennzeichnet ist durch:

(a) Vorrichtung (15) zur tiefen Abkühlung des Sterilisiergases, auf eine Temperatur, die zur Verflüssigung des Sterilisiermittels und des Verdünnungsgases ausreicht, aber nict ausreicht zur Verflüssigung anderer gasförmiger Verunreinigungen, mit denen das Sterilisiergas vermischt ist.

(b) Vorrichtung (128) zum Leiten der gasförmigen Verunreinigungen aus der Kühlvorrichtung (15) und zum Ausströmenlassen (101) der gennanten Verunreinigungen aus der Einrichtung und .

(c) Vorrichtung (130) zum Transport des verflüssigten Sterilisiermittels und des verflüssigten Verdünnungsgases aus der Einrichtung.

13. Einrichtung gemäß Anspruch 12, gekennzeichnet durch eine Vorrichtung (68) zum abtrennen des verflüssigten Sterilisiermittels von dem verflüssigten Verdünnungsgas, wobei die Trennungsvorrichtung (68) mit der Transportvorrichtung (130) verbunden ist.

14. Einrichtung gemäß Anspruch 12, gekennzeichnet durch eine Vorrichtung (71) zum Verdampfen des verflüssigten Sterilisiermittels und Verdünnungsgases zur Herstellung einer gereinigten Mischung von Sterilisiermittel und Verdünnungsgas une eine Leitungsvorrichtung (131, 77) um die genannte Mischung in die Sterilisierkammer zu leiten.

15. Einrichtung gemäß Anspruch 12, gekennzeichnet durch eine mit der Leitungsvorrichtung (131) verbundene Vorrichtung (132, 79) zur Zugabe zusätzlichen Sterilisiermittels zur gereinigten Mischung von Sterilisiermittel und Verdünnungsgas.

16. Einrichtung gemäß Anspruch 15, gekennzeichnet durch eine Vorrichtung (85) zur Messung der Konzentration des Sterilisiermittels in der Leitungsvorrichtung (131) und eine Vorrichtung (87) zur Regelung der Strömung zusätzlichen Sterilisiermittels aus einer Versorgung (79) mit einem solchen Sterilisiermittel in die Leitung (131).

17. Einrichtung gemäß Anspruch 12, gekennzeichnet durch eine Abtransportvorrichtung (129), die eine Filtervorrichtung (45, 47) zur Reinigung des durch die Abtransportvorrichtung strömenden Gases enthält.

18. Einrichtung gemäß Anspruch 17, gekennzeichnet durch eine mit der Abtransportvorrichtung (129) verbundene Vorrichtung (85) zur Messung des in dem ausströmenden Gas vorhandenen Sterilisiermittels und zum Signalisieren, daß die Filter (45, 47) ausgewechselt werden müssen.

19. Einrichtung gemäß Anspruch 18, dadurch gekennzeichnet, daß es mindestens zwei Filtervorrichtungen (45, 47) gibt, die parallelgeschaltet sind und daß die Meßvorrichtung (132) eine Vorrichtung (49) zum Umschalten von einem Filter (45) auf den anderen Filter (47) steuert, wenn die Menge an Sterilisiermittel im ausströmenden Gas einen festgelegten Wert überschreitet.

20. Einrichtung zur Behandlung eines Sterilisiergases, wobei das Sterilisiergas zum Sterilisieren des Inhalts einer Sterilisierkammer verwendet wurde, gekennzeichnet durch eine Vorrichtung (15) zur Kühlung des Sterilisiergases, so daß das Sterilisiergas verflüssigt und von gasförmigen Verunreinigungen im Sterilisiergas getrennt wird, eine Vorrichtung (129) zum Entlüften der gasförmigen Verunreinigungen aus der Kühlvorrichtung und eine Vorrichtung (130) zum Abtransport des verflüssigten Sterilisiergases aus der Einrichtung.

21. Einrichtung gemäß Anspruch 20, gekennzeichnet durch eine Vorrichtung (71) zum Verdampfen des verflüssigten Sterilisiergases und durch eine Vorrichtung (131, 77) zur Leitung des verdampften Sterilisiergases in die Sterilisierkammer.

22. Einrichtung gemäß Anspruch 20, dadurch gekennzeichnet, daß das Sterilisiergas aus einer Mischung besteht, die ein Sterilisiermittel und ein inertes Verdünnungsgas enthält, und daß die Kühlvorrichtung (15) zur Verflüssigung sowohl des Sterilisiermittels als auch des Verdünnungsgases ausgebildet ist.

23. Einrichtung gemäß Anspruch 20, dadurch gekennzeichnet, daß die Kühlvorrichtung (15) eine Vorrichtung (35) zur Leitung einer Tieftemperaturflüssigkeit in die unmittelbare Nähe des Sterilisiermittels und des Verdünnungsgases enthält, mittels der das Sterilisiermittel und das Verdünnungsgas verflüssigt wird.

24. Einrichtung gemäß Anspruch 20, worin die Entlüftungsvorrichtung (129) eine Filtervorrichtung (45, 47) zur Reinigung des durch die Entlüftungsvorrichtung (129) ausströmenden Gases enthält.

25. Einrichtung gemäß Anspruch 24, gekennzeichnet durch mindestens zwei Filtervorrichtungen (45, 47) wobei das ausströmende Gas jeweils nur durch eine Filtervorrichtung geleitet wird.

26. Einrichtung gemäß Anspruch 25, gekennzeichnet durch eine Vorrichtung (132) zum Überwachen der Konzentration des Sterilisiermittels das in dem durch die Entlüftungsvor richtung (129) ausströmenden Gas vorhanden ist, wobei die Überwachungsvorrichtung (132) verbunden ist mit einer Vorrichtung (49) zum Umschalten der Filtervorrichtungen (45, 47) durch die das Gas strömt, wenn die Konzentration des Sterilisiermittels, in dem ausströmenden Gas, einen festgelegten Wert überschreitet.

27. Einrichtung gemäß Anspruch 21, gekennzeichnet durch eine Vorrichtung (79, 81, 93) zur Zugabe von zusätzlichem Sterilisiermittel zur verdampften Mischung in der Leitung (131).

28. Einrichtung gemäß Anspruch 27, gekennzeichnet durch eine Vorrichtung (85) zur Messung der Konzentration des Sterilisiermittels in der Leitung (131), wobei die Meßvorrichtung (85) zur Regelung mit der Zugabevorrichtung (79, 81, 93) verbunden ist, so daß die Konzentration des Sterilisiermittels in der Leitung (131) im wesentlichen auf einem gewünschten Wert gehalten wird.

29. Verfahren zur Rückgewinnung einer oder mehrerer Komponenten eines Sterilisiergases, wobei das Sterilisiergas ein Sterilisiermittel und ein inertes Verdünnungsgas enthält, gekennzeichnet durch die folgenden Schritte :

(a) Kühlen des Sterilisiergases, so daß das Sterilisiermittel und das Verdünnungsgas verflüssigt wird,

(b) Filtrieren und Ausströmenlassen der gasförmigen Verunreinigungen, die nach dem Kühlen verbleiben,

(c) Filtrieren des verflüssigten Sterilisiergases,

(d) Verdampfen der filtrierten Flüssigkeit zur Herstellung eines gereinigten Sterilisiergases,

(e) Einstellen der Konzentration des Sterilisiermittels im Sterilisiergas durch die Zugabe von Sterilisiermittel, um die Konzentration auf einem Sollwert zu halten, und

(f) Rückführen der Mischung von Sterilisiermittel und Verdünnungsgas zur Sterilisierkammer.

30. Verfahren zur Behandlung eines Sterilisiergases, wobei das Sterilisiergas ein Sterilisiermittel und ein inertes Verdünnungsmittel enthält und gekennzeichnet ist durch die folgenden Schritte:

(a) Kühlen des Sterilisiergases, so daß das Sterilisiermittel und das Verdünnungsgas verflüssigt werden,

(b) Filtrieren und Ausströmenlassen der gasförmigen Verunreinigungen, die nach dem Kühlen zurückbleiben, und

(c) Rückgewinnung der Flüssigkeit nach dem Filtrieren und Ausströmen.

31. Verfahren gemäß Anspruch 30, gekennzeichnet durch das Filtrieren des verflüssigten Sterilisiergases.

32. Verfahren gemäß Anspruch 31, gekennzeichent durch das Verdampfen der filtrierten Flüssigkeit zur Herstellung eines gereinigten Sterilisiergases, durch das Einstellen der Konzentration des Sterilisiermittels im Sterilisiergas durch die Zugabe von Sterilisiermittel, um die Konzentration auf einem Sollwert zu halten und das Rückleiten der Mischung von Sterilisiermittel und Verdünnungsgas zur Sterilisierkammer.

33. Verfahren gemäß Anspruch 30, dadurch gekennzeichnet, daß das Kühlen durch die Leitung einer Tieftemperaturflüssigkeit in die unmittelbare Nähe des Sterilisiermittels und des Verdünnungsgases erfolgt.

34. Verfahren gemäß Anspruch 30, dadurch gekennzeichnet, daß die Durchleitung der gasförmigen Verunreinigungen nur durch einen von mehereren Filtern erfolgt.

35. Verfahren gemäß Anspruch 34, gekennzeichnet durch Überwachung der Konzentration des Sterilisiermittels in der verdampften Mischung und durch Zugabe von Sterilisiermittel zur verdampften Mischung in einer Menge, die ausreicht, um die Konzentration des Sterilisiermittels auf einem Sollwert zu halten.

36. Verfahren gemäß Anspruch 35, dadurch gekennzeichnet, daß das Kühlen durch den Transport einer Tieftemperaturflüssigkeit in die unmittelbare Nähe des Sterilisiermittels und des Verdünnungsgases erfolgt, so daß Wärme vom Sterilisiermittel und vom Verdünnungsgas auf die Tieftemperaturflüssigkeit übertragen werden kann.

37. Verfahren gemäß Anspruch 30, gekennzeichnet durch die Abtrennung des verflüssigten Sterilisiermittels von dem verflüssigten Verdünnungsmittel.

GAS TO STERILIZER

GAS FROM STERILIZER

GAS TO VENT

FILTER

HEATER

SWITCH

GAS CHROMATOGRAPH

FRESH GAS

EP 0 326 985 A2